(19) **Europäisches Patentamt / European Patent Office / Office européen des brevets**

(11) **EP 3 061 523 A1**

(12) **EUROPEAN PATENT APPLICATION**
published in accordance with Art. 153(4) EPC

(43) Date of publication:
**31.08.2016 Bulletin 2016/35**

(21) Application number: **14856142.6**

(22) Date of filing: **22.10.2014**

(51) Int Cl.:
**B01J 20/02** (2006.01)      **A23L 3/3436** (2006.01)
**B01D 53/14** (2006.01)      **B22F 1/00** (2006.01)
**C22C 21/00** (2006.01)      **C22C 21/12** (2006.01)

(86) International application number:
**PCT/JP2014/078132**

(87) International publication number:
**WO 2015/060356 (30.04.2015 Gazette 2015/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.10.2013   JP 2013219096**

(71) Applicant: **Mitsubishi Gas Chemical Company, Inc.**
**Tokyo 100-8324 (JP)**

(72) Inventors:
- **KAWAI Ryuichiro**
  **Tokyo 125-8601 (JP)**
- **TANAKA Hirokazu**
  **Tokyo 125-8601 (JP)**

(74) Representative: **Hoffmann Eitle**
**Patent- und Rechtsanwälte PartmbB**
**Arabellastraße 30**
**81925 München (DE)**

(54) **METHOD FOR STORING OXYGEN ABSORBER**

(57)     The method for storing an oxygen absorbing agent according to the present invention comprises: providing an oxygen absorbing agent comprising a metal, the metal having been obtained by subjecting an alloy comprising (A) at least one transition metal selected from the group consisting of iron, cobalt, nickel and copper and (B) aluminum to treatment with a basic aqueous solution to elute and remove at least a part of the aluminum (B), the oxygen absorbing agent having a specific surface area of at least 10 $m^2$/g as measured by a BET method; and immersing the oxygen absorbing agent in an aqueous solution containing a chelating agent to store the oxygen absorbing agent.

**Description**

Technical Field

The present invention relates to a method for storing an oxygen absorbing agent. More specifically, the present invention relates to a method for storing an oxygen absorbing agent that can absorb and remove oxygen even in a low-humidity atmosphere.

Background of the Invention

One of techniques for preserving foods, pharmaceutical products and the like is preservation utilizing oxygen absorbing agents (oxygen scavengers). Specifically, the preservation utilizing oxygen absorbing agents is a technique in which an oxygen scavenger capable of absorbing oxygen in atmosphere, together with an object, is placed within a hermetically sealable packaging body, and the inside of the hermetically sealable packaging body is brought to an oxygen-free state to prevent an oxidation-derived deterioration, a fungal deterioration, discoloration and the like of objects.
Oxygen scavengers formed of various inorganic materials and oxygen scavengers formed of various organic materials have hitherto been proposed for the removal of oxygen in atmosphere. Amongst others, a metal obtained by removing aluminum from an alloy composed of aluminum and iron or an alloy composed of aluminum and nickel using an aqueous sodium hydroxide solution has been used to develop an oxygen absorbing agent that can absorb and remove oxygen in an atmosphere even in a moisture-free or substantially moisture-free atmosphere having a humidity of not more than 30% RH (25°C), on the same level as conventional oxygen scavengers (for example, WO 2012/105457).
Also, WO 2012/105457 discloses that the oxygen absorbing agent composed of a metal, which has been obtained as described above, is immersed and stored in an aqueous acidic solution or an aqueous solution having buffering function, whereby the oxygen absorbing agent can be stably stored for a long period of time while maintaining the oxygen absorption activity of the oxygen absorbing agent.

PRIOR ART DOCUMENTS

Patent documents

Patent document 1: WO 2012/105457

[SUMMARY OF THE INVENTION]

Problem to Be Solved by the Invention

However, the oxygen absorbing agent described in the above patent document, especially, oxygen absorbing agent having high oxygen absorption performance is sensitively reacted with the aqueous acidic solution, and thus, even when the oxygen absorbing agent is immersed and stored in an aqueous acidic solution or an aqueous solution having buffering function, the oxygen absorbing agent cannot be stored while maintaining sufficient oxygen absorption performance, in some cases.
An object of the present invention is to solve the above problem with the prior art and to provide a method for storing an oxygen absorbing agent that can absorb oxygen in the atmosphere even in a moisture-free or substantially moisture-free atmosphere, and is highly safe, is low in cost, and can stably store the oxygen absorbing agent for a long period of time while maintaining sufficient oxygen absorption performance.

Means for Solving the Problem

The present inventors have noticed that, due to the treatment with a base such as sodium hydroxide for elution of the component (B) in the production of the oxygen absorbing agent, a metal hydroxide such as sodium hydroxide stays within pores formed in the metal formed by the elution of the component (B) and cannot be fully removed after the step of water washing, and that this residue is gradually eluted in water during storage of the oxygen absorbing agent in water. Further, the present inventors have inferred that, upon storage of the oxygen absorbing agent in the aqueous solution, the metal hydroxide such as sodium hydroxide eluted in the aqueous solution may be deposited on the surface of the oxygen absorbing agent with the elapse of time, leading to reduced oxygen absorption activity of the oxygen absorbing agent. As a result of further studies, it has been found that, even when the metal hydroxide is immersed in the aqueous solution, the addition of a chelating agent to the aqueous solution ensures storage of the oxygen absorbing agent while maintaining the oxygen absorption activity of the oxygen absorbing agent. The present invention has been made based on such finding.

The present invention provides the following [1] to [4].

[1] A method for storing an oxygen absorbing agent, the method comprising:

providing an oxygen absorbing agent comprising a metal, the metal having been obtained by subjecting an alloy comprising

(A) at least one transition metal selected from the group consisting of iron, cobalt, nickel and copper and
(B) aluminum

to treatment with a basic aqueous solution to elute and remove at least a part of the aluminum (B),
the oxygen absorbing agent having a specific surface area of at least 10 $m^2/g$ as measured by a BET method; and
immersing the oxygen absorbing agent in an aqueous solution containing a chelating agent to store the oxygen absorbing agent.

[2] The method for storing an oxygen absorbing agent according to [1], wherein the chelating agent is a salt of a hydroxycarboxylic acid.

[3] The method for storing an oxygen absorbing agent according to [1] or [2], wherein the content of the chelating agent in the aqueous solution is 0.1 part to 20 parts by weight based on 100 parts by weight of the oxygen absorbing agent.

[4] The method for storing an oxygen absorbing agent according to any one of [1] to [3], wherein the aqueous solution has a pH value of 7 to 12.

Effect of the Invention

Further, the method for storing an oxygen absorbing agent according to the present invention can allow the oxygen absorbing agent to be stably stored from just after the production thereof, can allow the oxygen absorbing agent to maintain the same oxygen absorption activity as that just after the production thereof even after long-term storage, and can allow the oxygen absorbing agent to absorb and remove oxygen in an atmosphere even in a moisture-free or substantially moisture-free atmosphere, on the same level as conventional oxygen scavengers.

[MODE FOR CARRYING OUT THE INVENTION]

Hereinafter, the embodiments of the present invention are illustrated. The following embodiments are given by way of example to explain the present invention, and the present invention is not limited to them.

The method for storing an oxygen absorbing agent according to the present invention comprises: providing an oxygen absorbing agent; and immersing the oxygen absorbing agent thus prepared in an aqueous solution containing a chelating agent to store the oxygen absorbing agent. Hereinafter, the respective steps are described in detail.

<Step of providing oxygen absorbing agent>

The oxygen absorbing agent according to the present embodiment comprises a metal, the metal having been obtained by containing an alloy comprising the following two components: (A) at least one transition metal selected from the group consisting of iron, cobalt, nickel and copper and (B) aluminum with a basic aqueous solution to elute and remove at least a part of the aluminum (B). The term "oxygen absorbing agent" as used herein refers to an oxygen absorbing agent that can selectively absorb oxygen from an atmosphere around a place where the oxygen absorbing agent has been installed.

The transition metal usable as the component (A) is selected from iron, cobalt, nickel and copper. The transition metals described above may be used either solely or in a combination of two or more of them. For example, when iron and nickel are selected, an Fe-Ni alloy may be used as the component (A). The component (A) is preferably iron or nickel, more preferably iron. Among them, iron is preferred because of high safety and low cost. The component (B) constituting the oxygen absorbing agent is aluminum.

The alloy used in the present embodiment comprises the component (A) and the component (B) as described above, and may further comprise molybdenum, chromium, titanium, vanadium, tungsten and the like as additive metals. The alloy may further comprise additive components such as cyanic acids.

The alloy comprising the component (A) and the component (B) as described above may be prepared by a melting method. Regarding the composition ratio of the component (A) and the component (B), preferably, when the proportion of the component (A) is 20 to 80% by weight, the proportion of the component (B) is 20 to 80% by weight.

More preferably, when the proportion of the component (A) is 30 to 70% by weight, the proportion of the component (B) is 30 to 70% by weight. More specifically, for example, when the component (A) is iron or nickel, preferably, the proportion of iron or nickel is 30 to 55% by weight while the proportion of aluminum is 45 to 70% by weight.

The alloy as such may be subjected to treatment with a basic aqueous solution. In general, the alloy is finely ground before the treatment with the basic aqueous solution. The term "alloy" as used herein refers to an alloy having a single composition that has a specific crystal structure, as well as an alloy mixture or a mixture of metals per se.

The alloy may be finely ground by a method properly selected from commonly used metal crushing/grinding methods. An example of the finely grinding method is one in which the alloy is ground by a jaw crusher, a roll crusher, a hammer mill or the like, and, if necessary, fine grinding with a ball mill is further performed. Alternatively, a method may also be adopted in which a molten metal of the alloy is finely ground by rapid solidification such as atomization. When atomization is adopted, fine grinding in an inert gas such as an argon gas is preferred. The atomization may be performed by a method described, for example, in Japanese Patent Application Laid-Open No. 23597/1993.

The particle diameter of the alloy powder is preferably in the range of 5 to 200 $\mu$m. The particle size distribution is preferably as narrow as possible. Sieving (classification) with commercially available sieves (for example, 200-mesh) may be properly performed from the viewpoints of removing large particles and providing uniform particle size distribution. The atomization is likely to provide near spherical powder particles and, at the same time, to provide a narrow particle size distribution. The average particle diameter of the alloy powder can be measured with a particle size/shape distribution measuring device.

The alloy or alloy powder obtained as described above is treated with a basic aqueous solution to elute and remove at least a part of the component (B). That is, a metal obtained by eluting and removing at least a part of the component (B) from the alloy is used as the oxygen absorbing agent used in the storing method according to the present invention. Any basic aqueous solution can be used with no particular limitation as long as the basic aqueous solution is one that does not dissolve or hardly dissolves the component (A), but on the other hand, that can dissolve and remove the component (B), i.e., that can leach out (elute) the component (B) from the alloy. Examples of bases usable in the basic aqueous solution include hydroxides of alkali metals such as sodium hydroxide and potassium hydroxide; hydroxides of alkali earth metals such as calcium hydroxide and magnesium hydroxide; carbonates of alkali metals such as sodium carbonate and potassium carbonate; and ammonia. In these basic aqueous solutions, a combination of two or more of the bases may be used, if necessary.

In the present embodiment, the basic aqueous solution is preferably an aqueous solution of a hydroxide of an alkali metal or alkali earth metal, more preferably an aqueous solution of sodium hydroxide. For example, the use of an aqueous sodium hydroxide solution as the basic aqueous solution is advantageous in that the removal of excess sodium hydroxide by water washing and the removal of eluted aluminum are easy and, thus, the effect of reducing the necessary times of water washing can be expected.

In the treatment with the basic aqueous solution, for alloy powder, it is common practice to introduce the alloy powder little by little into a basic aqueous solution with stirring. Alternatively, a method may be adopted in which the alloy powder is previously placed in water and a concentrated basic aqueous solution is added dropwise to the water containing the alloy powder.

In the treatment with the basic aqueous solution, the concentration of the basic aqueous solution is, for example, 5 to 60% by weight. More specifically, when sodium hydroxide is used, the concentration is preferably 10 to 40% by weight.

In the treatment with the basic aqueous solution, the temperature of the aqueous solution is, for example, preferably approximately 20 to 120°C, more preferably 25 to 100°C.

The treatment time for the treatment of the alloy or alloy powder with the basic aqueous solution may vary depending, for example, upon the shape, state, and amount of the alloy used, the concentration of the basic aqueous solution, but the temperature of the treatment is generally approximately 30 to 300 min. The amount of the component (B) eluted from the alloy may be regulated by regulating the treatment time.

In the present embodiment, at least a part of the component (B) is eluted and removed from the alloy by the treatment with the aqueous solution. Here eluting and removing "at least a part of the component (B)" means the elution and removal of a part of the component (B) from the alloy comprising the component (A) and the component (B), as well as the elution and removal of the whole component (B) from the alloy comprising the component (A) and the component (B). It cannot be denied that there is possibility that, in the process of eluting the component (B), a part of the component (A) is dissolved in the basic aqueous solution. Accordingly, "at least a part of the component (B)" is not limited to the elution of only the component (B) by the treatment with the basic aqueous solution.

At least a part, preferably a large proportion, of the component (B) is eluted from the alloy by the treatment with the basic aqueous solution. The proportion of the elution of the component (B) from the alloy can be expressed in terms of the content (residual ratio on a weight basis) of the component (B) in the metal obtained after the elution and removal.

The content of the component (B) in the metal used as the oxygen absorbing agent (that is, the metal after the elution of the component (B)) is preferably 0.1 to 60% by weight, more preferably 1 to 40% by weight. More specifically, for example, when the alloy is an Al-Fe alloy, the content of aluminum in the metal obtained by eluting and removing aluminum by the treatment with the basic aqueous solution from the alloy is preferably 0.1 to 50% by weight, more preferably 1 to 40% by weight, still more preferably 1 to 5% by weight. The content of the component (B) in the metal used in the oxygen absorbing agent may be measured, for example, by an ICP method.

The treatment with the basic aqueous solution may cause formation of a metal hydroxide of the component (A) and/or the component (B) on the alloy surface, in some cases. In order to improve the oxygen absorption performance of the oxygen absorbing agent, the metal hydroxide can be removed from the alloy. Specifically, after the elution of the component (B) from the alloy, the metal hydroxide can be washed with a basic aqueous solution having a higher concentration for removal.

The metal obtained as described above has a porous shape (or is in a porous body form). The porous shape refers to such a state that a number of pores having a size observable under an electron microscope are present on the surface or in the inside of the metal. In the present invention, the porosity in the porous shape of the metal can be expressed in terms of specific surface area. Specifically, the specific surface area of the metal used in the oxygen absorbing agent according to the present invention is at least 10 $m^2$/g, preferably at least 20 $m^2$/g, more preferably at least 40 $m^2$/g, further preferably at least 80 $m^2$/g, as measured by a BET method.

For example, in the present embodiment, when iron is used as the component (B), the specific surface area (measured by the BET method) of the resultant porous metal is approximately 10 to 120 $m^2$/g, whereas a nonporous conventional iron powder (reduced iron powder or atomized iron powder) has a specific surface area of approximately 0.07 to 0.13 $m^2$/g, demonstrating that the former is porous.

The porosity in the porous shape of the metal may also be expressed in terms of bulk density. The bulk density of the oxygen absorbing agent (metal) obtained in the above manner is not more than 2 $g/cm^3$, preferably not more than 1.5 $g/cm^3$. Incidentally, the nonporous conventional iron powder (reduced iron powder or atomized iron powder) has a bulk density of approximately 2 to 3 $g/cm^3$. The bulk density can be measured according to JIS Z 2504.

In the present embodiment, the porous metal used in the oxygen absorbing agent has a high level of oxygen absorption activity and thus may also be of course suitable for use as an oxygen absorbing agent even under an atmosphere having a low humidity (for example, 30% RH or less (relative humidity) (25°C)). It is needless to say that the porous metal is also suitable as oxygen abosrbing agents even under high-humidity conditions (for example, under 100% RH (relative humidity) (25°C) conditions.

Accordingly, the metal obtained as described above can absorb at least 5 mL/g of oxygen, more preferably 10 mL/g of oxygen under an atmosphere having a low humidity of 30% RH or less (relative humidity) (25°C). When the metal is used as the oxygen absorbing agent, the amount of oxygen absorbed is 5 to 150 mL/g under an atmosphere having a low humidity of 30% RH or less (relative humidity) (25°C).


< Method for storing oxygen absorbing agent >

After the treatment for the elution and removal of the component (B), in general, the metal is normally washed with water. The metal or metal powder thus obtained generally immediately undergoes a deterioration by oxidation in the air. Accordingly, after the treatment of the alloy comprising the component (A) and the component (B) with the basic aqueous solution, caution is required so as to minimize the contact of the metal and the alloy with oxygen. To meet this requirement, the practice of a series of treatments in an aqueous solution or water to obtain a treated metal which as such is stored in the aqueous solution or water, or storage under an oxygen-free atmosphere or under an inert gas atmosphere is considered effective. However, it has been found that the storage of the metal in water causes the oxygen absorption activity to be lowered with the elapse of time although a rapid lowering in the oxygen absorption activity can be prevented. The storage under an oxygen-free atmosphere or under an inert gas atmosphere disadvantageously incurs an increased cost due to the necessity of apparatuses or the like.

In the present invention, the storage of the oxygen absorbing agent formed of the metal by immersing in a specific aqueous solution containing a chelating agent can realize stable storage after the production of the oxygen absorbing agent, can allow the oxygen absorption activity on the same level as that just after the production of the oxygen absorbing agent to be maintained even after long-term storage, and can allow oxygen in an atmosphere to be absorbed and removed on the same level as conventional oxygen scavengers even in a moisture-free or substantially moisture-free atmosphere.

Although the reason why the oxygen absorption activity of the oxygen absorbing agent can be maintained by using an aqueous solution containing a chelating agent in the present invention is unclear, the following mechanism, for example, is inferred. Specifically, due to the treatment with a base such as sodium hydroxide for elution of the component (B) in the production of the oxygen absorbing agent as described above, sodium hydroxide or the like stays within pores formed in the metal formed by the elution of the component (B) and cannot be fully removed after the step of water washing, and the metal hydroxide residue is gradually eluted in water during storage of the oxygen absorbing agent in water. Thereafter, an aluminum-containing compound may be deposited on the surface of the oxygen absorbing agent to occlude the active point of oxygen absorption. However, the use of an aqueous solution containing a chelating agent allows the chelating agent to block aluminum ions. It is inferred that this would prevent occlusion of the active point by the deposited product.

Water is suitable as the storage medium in which the oxygen absorbing agent is immersed, from the viewpoints of safety and cost. Accordingly, an aqueous solution containing a chelating agent is used as the aqueous solution in which the oxygen absorbing agent is immersed. The pH value of the aqueous solution is preferably in the range of 7 to 12, more preferably 8 to 12, further preferably 10 to 11. When the pH of the aqueous solution is defined within the above range, the dissolution of the iron (Fe) component in the oxygen absorbing agent can be further suppressed.

Substances usable as the chelating agent as described above are not particularly limited, and at least one or two or more selected from salts of hydroxycarboxylic acids such as glycolic acid, lactic acid, malic acid, tartaric acid, citric acid, dalconic acid (*sic.*) and heptonic acid; salts of dicarboxylic acids such as succinic acid, malonic acid, oxalic acid and phthalic acid; salts of polycarboxylic acids such as polyacrylic acid and polymaleic acid; aminocarboxylic acids such as glycine, nitrilotriacetic acid, ethylenediamine tetraacetic acid (EDTA), hydroxyethylethylenediamine triacetic acid (HEDTA), diethylenetriamine pentaacetic acid and polyamino polycarboxylic acid and their salts; phosphorous acid-based chelating agents such as etidronic acid (HEDP), nitrilotris(methylenephosphonic acid) (ATMP) and ethylenediamine tetramethylene phosphonic acid (EDTMP) and their salts; amine-based chelating agents such as ethylenediamine, diethylenetriamine and triethylenetetramine can be used in combination. Among these, salts of hydroxycarboxylic acids are preferred, alkali metals of hydroxycarboxylic acids are more preferred, sodium lactate, sodium malate, sodium tartrate, sodium citrate and sodium gluconate are further preferred, and sodium tartrate, sodium citrate and sodium gluconate are particularly preferred, from the viewpoints of safety for human bodies and cost.

A chelate resin can also be suitably used as the chelating agent used in the present invention. Examples of substances usable as the chelate resin include Amberlite IRC748 (manufactured by ORGANO CORPORATION) and DIAION CR11 (manufactured by Mitsubishi Chemical Corporation).

Also, in the method for storing an oxygen absorbing agent according to the present embodiment, the content of the chelating agent in the aqueous solution is preferably in the range of 0.1 part to 20 parts by weight, more preferably 1 part to 10 parts by weight, particularly preferably 2 parts to 5 parts by weight based on 100 parts by weight of the oxygen absorbing agent. The concentration of the chelating agent is defined within the above range, thereby ensuring further maintenance of the oxygen absorption activity just after the production of the oxygen absorbing agent.

In the present embodiment, preferably, the oxygen absorbing agent is immersed and stored at a temperature equal to or lower than room temperature in an aqueous solution in an amount of not less than twice the weight of the oxygen absorbing agent. The amount of the aqueous solution is defined as not less than twice the weight of the oxygen absorbing agent, whereby the oxygen absorbing agent can be satisfactorily immersed in the aqueous solution

< Method of using oxygen absorbing agent >

In use of the oxygen absorbing agent stored in the aqueous solution containing the chelating agent, the oxygen absorbing agent may be taken out from the aqueous solution followed by drying. However, as described above, the oxygen absorbing agent is likely to be deteriorated by oxidation in the air. Accordingly, from the viewpoint of preventing a lowering in oxygen absorption activity, for example, a method is preferably adopted in which, before use, the oxygen absorbing agent is dried under such conditions that the influence of oxygen is removed as much as possible, for example, by vacuum drying.

Since the oxygen absorbing agent according to the present invention is likely to be deteriorated by oxidation in the air as described above, in use, the oxygen absorbing agent can be mixed (kneaded) in a resin and used in the form of the resultant oxygen-absorbing resin.

While the kinds of usable resins are not particularly limited, thermoplastic resins such as a polyolefin resin, a polyester resin, a polyamide resin, a polyvinyl alcohol resin and a chlorine-based resin can be used. Especially, polyethylene, polypropylene, ethylene-vinyl acetate copolymers, elastomers or mixtures thereof can be suitably used.

In order to store commodities having a low water content that should be stored under low-humidity conditions, the relative humidity (RH) of an atmosphere in which the commodities having a low water content are stored is preferably 20 to 70%, more preferably 20 to 50%. The water content of commodities having a low water content is preferably not more than 50% by weight, more preferably not more than 30% by weight, particularly preferably not more than 10% by weight. Commodities having a low water content (products to be packaged) that should be stored under low-humidity conditions include, for example, foods and pharmaceutical products that are weak against an increase in water content and are required to avoid inclusion of foreign materials, for example, powdery and granular foods (powder soups, powder beverages, powder confectioneries, seasoners, grain powders, nutritional foods, health foods, artificial colors, flavoring agents, and spices and condiments), powdery and granular pharmaceutical products (medicinal powders, powdered soaps, toothpastes, and heavy chemicals), and molded products (tablets) thereof. In particular, when the products to be packaged are filled into oxygen absorbing packaging bodies which will be described later, oxygen in an atmosphere even in a moisture-free or substantially moisture-free atmosphere, can be absorbed and removed on the same level as that attained by conventional oxygen scavengers. Accordingly, the oxygen absorbing agent is suitable for use in applications where an atmosphere in packages of dried foods, pharmaceutical products, and electronic materials, which are weak against moisture and for which conventional oxygen scavengers cannot be applied, is brought to an oxygen-free state. The oxygen absorbing agent is suitable for use, for example, in dried foods such as powder seasoners, powder coffees, coffee beans, rices, teas, beans, baked rice chips, and rice crackers, pharmaceutical products and health foods such as vitamin preparations.

The oxygen absorbing packaged body according to a further embodiment of the present invention comprises: the above oxygen absorbing agent or oxygen absorbing resin composition; and a packaging material, the whole or a part of which is formed of an air-permeable packaging material, the oxygen absorbing agent or oxygen absorbing resin composition having been packaged into the packaging material. Examples of the packaging material include a packaging material prepared by laminating two air-permeable packaging materials and forming a bag from the laminate, a packaging material prepared by laminating one air-permeable packaging material and one air-impermeable packaging material and forming a bag from the laminate, or a packaging material prepared by folding one air-permeable packaging material and mutually sealing edges except for the folded part to form a bag. Packaging materials permeable to oxygen and carbon dioxide are usable as the air-permeable material. Examples of such air-permeable packaging materials include papers, nonwoven fabrics, and conventional plastic films that have been treated to render them permeable to air.

[EXAMPLES]

The present invention is further illustrated by the following Examples. However, the present invention is by no means to be construed as being limited to them.

< Production and evaluation of oxygen absorbing agent >

An Al (aluminum) powder (50% by weight) and an Fe (iron) powder (50% by weight) were mixed together and were dissolved in each other in nitrogen to obtain an Al-Fe alloy. Then, the Al-Fe alloy thus obtained was ground with a jaw crusher, a roll crusher, and a ball mill, and the ground product was classified with a net having an opening of 200 meshes (0.075 mm) to obtain an Al-Fe alloy having a particle size of not more than 200 meshes. The Al-Fe alloy powder (150 g) thus obtained was mixed while stirring for one hr in a 30% by weight aqueous sodium hydroxide solution at 50°C. The Al-Fe alloy powder was separated by filtration, and further mixed while stirring for one hr in a 40% by weight aqueous sodium hydroxide solution at 50°C. Sequentially, the mixed solution was allowed to stand, and the upper layer liquid was removed. The residual precipitate was washed with distilled water until pH became 11 or less to obtain a porous Al-Fe alloy powder. In order to avoid contact with oxygen, the porous alloy powder was prepared by a reaction in an aqueous solution in this manner.

The porous metal powder thus obtained was dried in vacuo under conditions of not more than 200 Pa and 80°C for 2 hr to obtain a dried product of a porous Al-Fe metal powder. The alloy powder thus obtained had a bulk density of 0.9 $g/cm^3$ (as measured according to JIS Z 2504). The alloy powder thus obtained (0.5 g) was packaged in an air-permeable small bag and, together with a desiccant, was placed in a gas barrier bag (an Al foil-laminated plastic bag). The gas barrier bag was filled with 50 mL of air (oxygen concentration: 20.9% by volume), was hermetically sealed, and was stored at 25°C for seven days. The concentration of oxygen after the storage at 25°C for seven days was measured and was found to be 9.1% by volume. The amount of oxygen absorbed was calculated from the reduced oxygen concentration within the gas barrier bag and was found to be 130 mL/g.

The average particle diameter of the porous Al-Fe alloy powder was measured with a particle size/shape distribution measuring device ("PITA-2," manufactured by Seishin Enterprise Co., Ltd.) and was found to be 31 $\mu$m. Further, the specific surface area of the porous Al-Fe alloy powder thus obtained was measured with an automatic specific surface area measuring device (GEMINI VII2390, manufactured by Shimadzu Seisakusho Ltd.) and was found to be 100 $m^2/g$.

Example 1

Sodium tartrate (2 g) was added as a chelating agent and stored in a water slurry (300 g) containing the oxygen absorbing agent (100 g) composed of the porous Al-Fe alloy powder obtained as described above. A part of the oxygen absorbing agent was sampled after elapse of 14, 28 and 56 days from the start of the storage, the oxygen absorbing agent was dried in vacuo to a water content of not more than 1% by weight as measured under conditions of not more than 200 Pa and 80°C. Thereafter, in the same manner as described above, the oxygen concentration was measured to calculate the amount of oxygen absorbed by the oxygen absorbing agent. Oxygen absorbing capability retention (%) was calculated from the oxygen absorption amount thus obtained by the following equation.

Oxygen absorbing capability retention (%) = (Oxygen absorption amount after the storage)/(Oxygen absorption amount just after the preparation = 130) × 100

The results were as shown in Table 1 below.

Example 2

The measurement was carried out in the same manner as in Example 1, except that the amount of sodium tartrate added in Example 1 was changed to 5 g, for calculation of the oxygen absorbing capability retention. The results of measurement were as shown in Table 1 below.

Example 3

The measurement was carried out in the same manner as in Example 1, except that sodium tartrate was replaced with sodium gluconate, for calculation of the oxygen absorbing capability retention. The results of measurement were as shown in Table 1 below.

Comparative Example 1

The measurement was carried out in the same manner as in Example 1, except that no sodium tartrate was added to the water slurry, for calculation of the oxygen absorbing capability retention. The results of measurement were as shown in Table 1 below.

Comparative Example 2

The measurement was carried out in the same manner as in Example 1, except that sodium tartrate was changed to citric acid, for calculation of the oxygen absorbing capability retention. The results of measurement were as shown in Table 1 below.

Comparative Example 3

The measurement was carried out in the same manner as in Example 1, except that a citric acid/sodium citrate buffer solution (100 g) having pH 6 prepared so as to have a concentration of 0.2 M was added and stored in a water slurry (200 g) containing the oxygen absorbing agent (100 g) composed of the porous Al-Fe alloy powder used in Example 1, for calculation of the oxygen absorbing capability retention. The results of measurement were as shown in Table 1 below.

[Table 1]

| | Solute of storing solution | | pH after addition | Oxygen absorbing capability retention (%) | | |
| | Kind | Added amount (part) | | After 14 days | After 28 days | After 56 days |
|---|---|---|---|---|---|---|
| Example 1 | Sodium tartrate | 2.0 | 10.9 | 91 | 91 | 80 |
| Example 2 | Sodium tartrate | 5.0 | 10.9 | 81 | 80 | 75 |
| Example 3 | Sodium gluconate | 2.0 | 10.9 | 86 | 76 | 76 |
| Comparative Example 1 | --- | --- | 11.0 | 11 | 11 | --- |
| Comparative Example 2 | Citric acid | 2.0 | 2.3 | 77 | 66 | --- |
| Comparative Example 3 | 0.2M Citric acid buffer solution | --- | 6.1 | 65 | 60 | --- |

It has been found that, in Examples 1 to 5 employing an aqueous solution containing a chelating agent as a storing solution, the oxygen absorbing capability retention after 28 days is a high value, 76% to 91%, so that the reduction in performance of the oxygen absorbing agent can be suppressed. On the other hand, it has been revealed that, in Comparative Examples 1 to 3 employing water, an aqueous citric acid solution or the like as a storing solution for the oxygen absorbing agent, the oxygen absorbing capability retention after 28 days ranges from 11% to 66%, so that the reduction in performance of the oxygen absorbing agent cannot be suppressed.

**Claims**

1. A method for storing an oxygen absorbing agent, the method comprising:

   providing an oxygen absorbing agent comprising a metal, the metal having been obtained by subjecting an alloy comprising

   (A) at least one transition metal selected from the group consisting of iron, cobalt, nickel and copper and
   (B) aluminum

   to treatment with a basic aqueous solution to elute and remove at least a part of the aluminum (B),
   the oxygen absorbing agent having a specific surface area of at least 10 m$^2$/g as measured by a BET method; and
   immersing the oxygen absorbing agent in an aqueous solution containing a chelating agent to store the oxygen absorbing agent.

2. The method for storing an oxygen absorbing agent according to claim 1, wherein the chelating agent is a salt of a hydroxycarboxylic acid.

3. The method for storing an oxygen absorbing agent according to claim 1 or 2, wherein the content of the chelating agent in the aqueous solution is 0.1 part to 20 parts by weight based on 100 parts by weight of the oxygen absorbing agent.

4. The method for storing an oxygen absorbing agent according to any one of claims 1 to 3, wherein the aqueous solution has a pH value of 7 to 12.

**EP 3 061 523 A1**

<table>
<tr><td colspan="2" align="center">**INTERNATIONAL SEARCH REPORT**</td><td>International application No.<br>PCT/JP2014/078132</td></tr>
</table>

A. CLASSIFICATION OF SUBJECT MATTER

*B01J20/02*(2006.01)i, *A23L3/3436*(2006.01)i, *B01D53/14*(2006.01)i, *B22F1/00* (2006.01)i, *C22C21/00*(2006.01)i, *C22C21/12*(2006.01)i

According to International Patent Classification (IPC) or to both national classification and IPC

B. FIELDS SEARCHED

Minimum documentation searched (classification system followed by classification symbols)
B01J20/02, A23L3/3436, B01D53/14, B22F1/00, C22C21/00, C22C21/12

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2012/105457 A1 (Mitsubishi Gas Chemical Co., Inc.),<br>09 August 2012 (09.08.2012),<br>claims; paragraphs [0048] to [0059]; examples 27, 29, 31, 33 to 35, 37, 38, 40<br>& JP 5246384 B2    & US 2013/0209350 A1<br>& EP 2606959 A1    & CN 103153452 A<br>& KR 10-2013-0045383 A    & TW 201247307 A | 1–4 |
| A | JP 9-253481 A (Sony Corp.),<br>30 September 1997 (30.09.1997),<br>entire text<br>(Family: none) | 1–4 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>14 January 2015 (14.01.15) | Date of mailing of the international search report<br>27 January 2015 (27.01.15) |
|---|---|
| Name and mailing address of the ISA/<br>Japan Patent Office | Authorized officer |
| Facsimile No. | Telephone No. |

Form PCT/ISA/210 (second sheet) (July 2009)

**INTERNATIONAL SEARCH REPORT**

International application No.

PCT/JP2014/078132

C (Continuation). DOCUMENTS CONSIDERED TO BE RELEVANT

| Category* | Citation of document, with indication, where appropriate, of the relevant passages | Relevant to claim No. |
|---|---|---|
| A | JP 54-99092 A (Daishiro FUJISHIMA), 04 August 1979 (04.08.1979), entire text (Family: none) | 1-4 |
| A | JP 56-2845 A (Mitsubishi Gas Chemical Co., Inc.), 13 January 1981 (13.01.1981), entire text (Family: none) | 1-4 |
| A | JP 62-244443 A (Kabushiki Kaisha Shimadaya Honten), 24 October 1987 (24.10.1987), entire text & US 4836952 A & EP 241917 A2 | 1-4 |
| P,X | WO 2014/021430 A1 (Mitsubishi Gas Chemical Co., Inc.), 06 February 2014 (06.02.2014), claims; paragraphs [0047] to [0048]; examples & TW 201420181 A | 1-4 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012105457 A

- JP 5023597 A